# EUROPEAN PATENT SPECIFICATION

(11) **EP 4 552 661 B1**
(45) Date of publication and mention of the grant of the patent: **14.10.2026**
(21) Application number: 25165417.4
(22) Date of filing: 13.07.2021
(51) Int. Cl.: A61L 29/04, A61L 29/08, A61L 29/14, A61L 29/16

(54) **MEDICAL DEVICES HAVING ANTI-MICROBIAL PROPERTIES AND METHODS FOR MAKING THE SAME**
MEDIZINISCHE VORRICHTUNGEN MIT ANTIMIKROBIELLEN EIGENSCHAFTEN UND VERFAHREN ZUR HERSTELLUNG DAVON
DISPOSITIFS MÉDICAUX AYANT DES PROPRIÉTÉS ANTIMICROBIENNES ET LEURS PROCÉDÉS DE FABRICATION

(30) Priority: 13.07.2020 US 202063050935 P
(43) Date of publication of application: 14.05.2025
(62) Document number(s) of the earlier application(s) in accordance with Art. 76 EPC: 21749508.4 / 4 178 637
(73) Proprietor: Hollister Incorporated, Libertyville, IL 60048 (US)
(72) Inventor: Murnaghan, Kevin, Libertyville, IL 60048 (US); Farrell, David J., Libertyville, IL 60048 (US)
(74) Representative: FRKelly

(56) References cited:
- US-A1- 2007 093 894
- NEOH KOON GEE ET AL: "Surface modification strategies for combating catheter-related complications: recent advances and challenges", vol. 5, no. 11, 1 January 2017 (2017-01-01), GB, pages 2045 - 2067, XP055850895, ISSN: 2050-750X, Retrieved from the Internet <URL:https://pubs.rsc.org/en/content/articlepdf/2017/tb/c6tb03280j> DOI: 10.1039/C6TB03280J

## Description

The present application claims the benefit of and priority to U.S. Provisional Application No. 63/050,935, filed July 13, 2020.

### Technical Field

The present disclosure is directed to lubricious medical devices that include an anti-microbial disposed on an outer surface of the medical device to enhance the anti-microbial properties of the medical device. More specifically, the present disclosure is directed to medical devices that include a sulfanilamide, substituted sulfanilamide and/or a sulfanilamide derivative disposed on the outer surface of the medical device.

### Background

Insertable medical devices such as catheters are used to treat and facilitate treatment for a wide variety of medical conditions. These insertable medical devices have a myriad of uses including opening bodily passageways, delivering drugs, and aiding surgeons in surgeries. For example, urinary catheters are used to treat urinary incontinence. Intermittent urinary catheters are often used for self-catherization in non-sterile environments. Because these devices are inserted into the body, sterility and maintaining sterility is advantageous in preventing infections.

In such devices, pathogens including bacteria and microbes may be present on the surface or, where applicable, the coating of the medical devices. These pathogens can contaminate the medical device, causing the sterility of the catheter to be compromised. This contamination may result in catheter users, many of whom may be at an increased risk for infection, developing an infection. Thus, there remains a need for medical devices which resist pathogenic contamination. Koon Gee Neoh et al. disclose surface modification strategies for indwelling medical devices by a surface-immobilized antimicrobial moiety. The covalent grafting of an antimicrobial agent onto surfaces is expected to provide longer term effects than the use of an eluting agent (Journal of Materials Chemistry B, vol. 5, no. 11, pages 2045-2067).

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In a first aspect, a medical device includes a surface, and an antimicrobial comprising at least one of a sulfanilamide or a diaminodiphenyl sulfone, wherein the antimicrobial is grafted to the surface.

Described herein, but not claimed, is a method of making a medical device having antimicrobial properties. The method includes reducing a diazonium sulfanilamide salt or a diazonium diaminodiphenyl sulfone salt in the presence of a reducing agent and a surface of the medical device whereby at least one of a sulfanilamide derivative or a diaminodiphenyl sulfone derivative attaches to the surface of the medical device.

### Brief Description of the Drawings

Fig. 1 is a perspective view of a medical device; and
Fig. 2 is a schematic view of a chemical process, showing formation of a sulfanilamide derivative and the attachment of the sulfanilamide derivative to a surface of the medical device of Fig. 1.

### Description of the Illustrated Embodiments

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

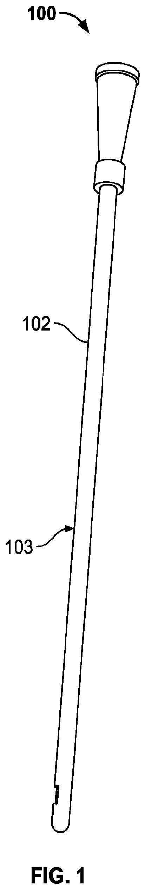

Fig. 1 shows an embodiment of a medical device 100, such as a urinary catheter. Although the subject matter disclosed herein may be described relative to urinary catheters, the subject matter is not limited to such, and such subject matter may apply to other suitable medical devices as well. The medical devices may be, for example, those which are configured for insertion into a lumen of a human body, such as the urethra, fallopian tubes, nasal passages or esophagus. Such medical devices may include, but are not limited to, catheters and endoscopes.

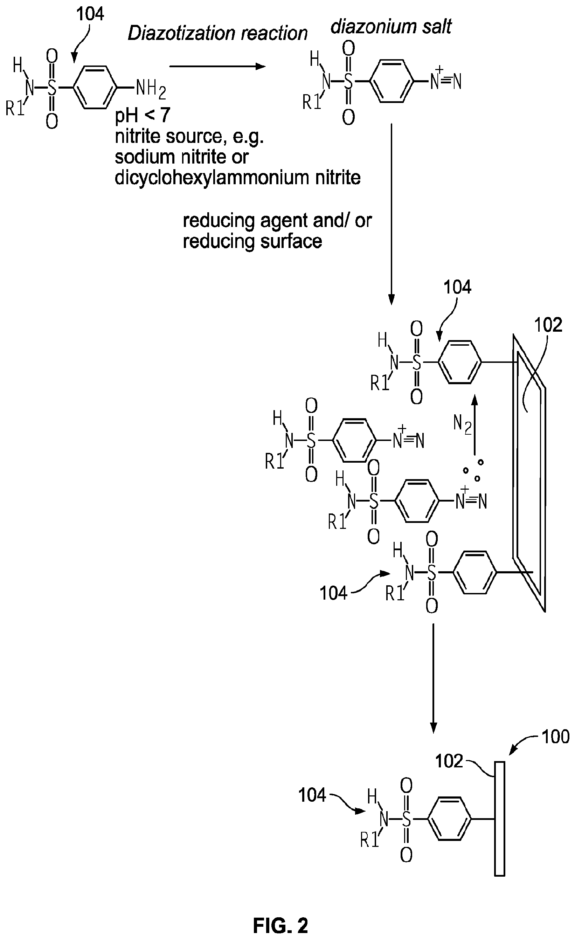

The medical device 100 comprises a surface 102. A sulfanilamide 104 (shown in Fig. 2) may be associated with the surface 102 and may be attached to the surface 102. In an embodiment, the sulfanilamide 104 may be attached to the surface 102 by grafting (i.e. chemical grafting). Grafting the sulfanilamide 104 to the surface 102 results in the surface having biocidal and antimicrobial properties. As used herein the term sulfanilamide(s) may encompass sulfanilamides, substituted sulfanilamides, and/or sulfanilamide derivatives.

Turning to Fig. 2, the sulfanilamide 104 may be attached to the surface 102 by reducing a diazonium sulfanilamide salt in the presence of a reducing agent and, also in the presence of the surface 102 of the medical device 100, thereby attaching the sulfanilamide (a sulfanilamide derivative) 104 to the surface 102 of the medical device 100. For example, the surface of a medical device may be placed in contact with a solution containing the diazonium sulfanilamide salt, a reducing agent, and an acid. In one alternative, the acid may be a Bronsted acid, such acids may include nitric acid, phosphoric acid, hydrochloric acid, sulfuric acid, tetrafluoroboric acid or hexafluorophosphoric acid. Some acids may not be suitable for and would not be used in the process described herein. Such acids include hexafluorometallic acids such as hexafluorozironic or hexafluorotitanic acid. In one embodiment, the pH of the solution may be less than 7, and may be for example, between 2 and 4.

The surface 102 of the medical device 100 may be a plastic or polymer surface. The reducing agent may include a metal, such as steel or aluminum. Alternatively, the reducing agent may be metallic powders including, but not limited to, steel, aluminum, iron, or zinc powder. In another alternative the surface of the device itself could be or could include the reducing agent. For example, the surface of the device could be a metal surface that acts as a reducing agent, or the surface could be a polymer that includes a metal reducing agent incorporated therein. In another alternative, organic reducing agents, such as but not limited to: acetyl phenyl hydrazine, tannic acid, benzoin, ascorbic acid (vitamin A), ascorbic acid 6-palmitate, alpha-tocopherol, gamma -tocopherol, 2,2,5,7,8-pentamethylchromanol, gallic acid [3,4,5-trihydroxybenzoic acid], pyrogallol [benzene-1 ,2,3-triol], hydroquinone and combinations thereof, may be used. The reducing agent may be formed in solution.

To form the diazonium sulfanilamide salt, the sulfanilamide 104 may undergo a diazotization reaction, as shown in the first reaction in the schematic of Fig. 2. The sulfanilamide 104 may be reacted with a nitrogen source, such as a nitrite, which may include but is not limited to sodium nitrite, and dicyclohexylammonium nitrite. The nitrogen source also may be nitrous acid, or other group I or group II salts of nitrous acid, such as potassium nitrite, or calcium nitrite. Additionally, any other suitable nitrogen source may be used. In one embodiment, the sulfanilamide and nitrogen source may be placed in a solution to form the diazonium salt.

The diazonium salt may be formed from a sulfanilamide such as: 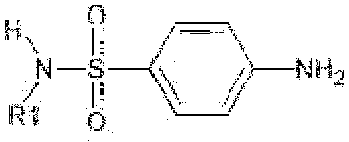 wherein R1 comprises one of (i) hydrogen, (ii) acetyl groups, (iii) amido groups, (iv) guanidyl groups, (v) 5 or 6 membered heterocyclic aromatic groups, (vii) methoxy-substituted heteroaromatic compounds and (vi) alkyl-substituted heteroaromatic compounds.

The 5 or 6 membered heterocyclic aromatic groups contain one or more of nitrogen atom(s), oxygen atom(s) or sulfur atom(s).
For example, the sulfanilamide 104 may be one or more of the below or derivatives thereof: 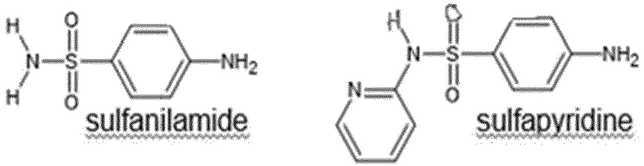 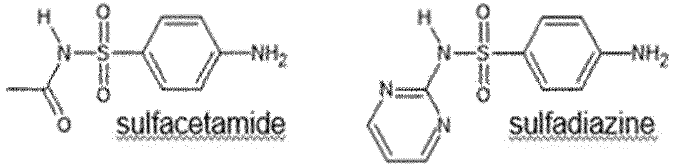 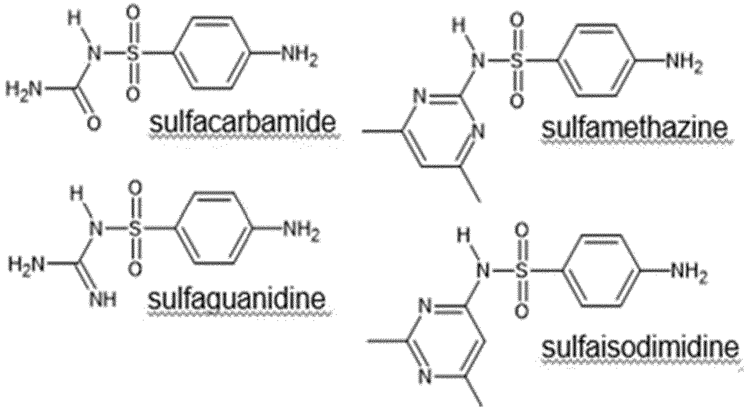 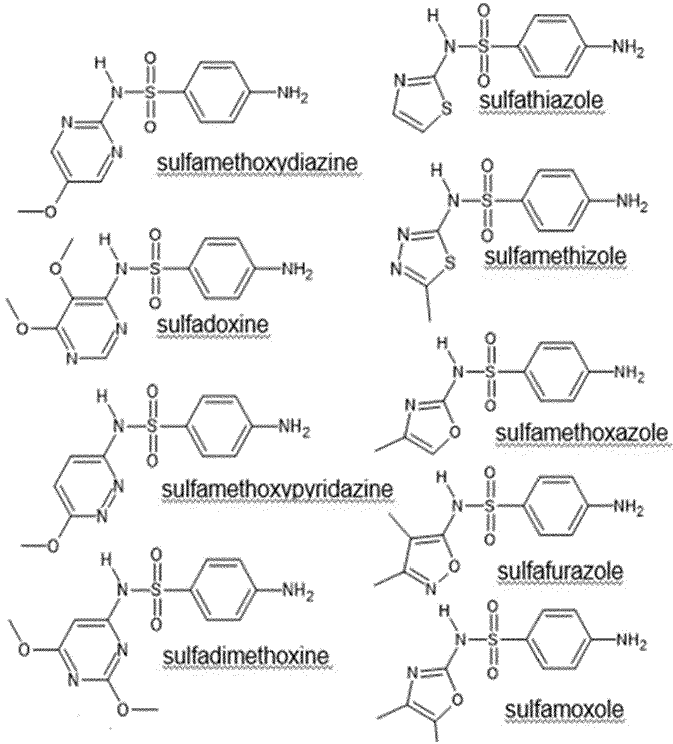 Other known suitable sulfanilamides besides those listed above may also be used. One of ordinary skill could readily understand that the above listed sulfanilamide and substituted sulfanilamides ("sulfanilamides") also represent corresponding derivatives of sulfanilamides. When attached to the medical device, the corresponding derivatives of the sulfanilamides have substantially the same structure as the above sulfanilamides except that the primary amine group of the sulfanilamides has been removed by reduction to facilitate attachment to the surface of the medical device. Reduction results in a covalently bound sulfanilamide attached to the surface of the medical device.

In an alternative to sulfanilamides, or in addition to, diaminodiphenyl sulfone (Dapsone) may be attached to the surface of a medical device using the same or similar mechanisms as disclosed herein. Diaminodiphenyl sulfone includes derivatives thereof, such as the diazonium forms of diaminodiphenyl sulfone. For example, diaminodiphenyl sulfone may be transformed into its mono or bis diazonium salt forms by the same or similar mechanisms as disclosed herein. The diazonium diaminodiphenyl sulfone salt may then be reduced in the presence of a medical device surface to attach diaminodiphenyl sulfone.

As shown in Fig. 2, reduction of the diazonium salt and the surface grafting process may result in stoichiometric amounts of nitrogen gas being released. The organic groups may also be bound to each other during the reduction process (coupling). Diazonium compounds may also be usable as coupling agents for further surface modification.

Thus, upon grafting, the sulfanilamide or derivative thereof 104 attached to the surface of the medical device may be any of the above mentioned sulfanilamides. In particular, the sulfanilamide or derivative thereof may be: 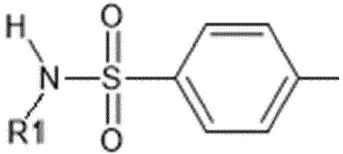 wherein R1 is any of the above-mentioned components or functional groups.

In an embodiment, the grafting of the sulfanilamide 104 to the surface 102 of the medical device 100 may result in a monolayer--a thin layer of organic groups grafted to the surface 102. In alternative embodiments, a plurality of layers may be grafted onto the medical device, for example a bilayer may be formed on the surface, resulting in a thicker layer of organic groups grafted onto the surface.

The medical device 100 may be a catheter. As illustrated in Fig. 1, the medical device 100 may be a urinary catheter. The catheter, such as the illustrated urinary catheter, may include a shaft 103 and the sulfanilamide 104 may be attached to the shaft 103. In an embodiment, the medical device surface may be bare plastic and the sulfanilamide may be grafted onto the plastic. The medical device 100 may be made of any appropriate material known to one of ordinary skill in the art including, but not limited to, low-density polyethylene (LDPE), TPE, PU, or other polymeric materials.

In alternative embodiments, the medical device may have a hydrophilic coating on a surface of the medical device. For example, a urinary catheter may have a hydrophilic coating disposed on the outer surface of the catheter. The sulfanilamide may be attached to the hydrophilic coating. For example, a lubricious hydrophilic polymer coating may be on the surface of the medical device. For example, the hydrophilic coating may be formed from polyvinylpyrrolidone or polyethylene oxide, which may optionally include agents and additives. In such an embodiment, the sulfanilamide may be grafted onto the hydrophilic coating. When the hydrophilic coating is contacted, wetted or hydrated with a hydration medium, it becomes lubricious. The lubricity eases introduction of the medical device into the body and aids in reducing pain and discomfort associated with such introduction. The hydrophilic coating can be a single layer or a multilayer hydrophilic coating. Multi-layered coating can include at least a base coat and a top layer. In other alternatives, coatings other than hydrophilic coatings may be on a surface of the medical device, wherein the sulfanilamide is attached to the coating.

The sulfanilamides described above are anti-bacterial, and anti-microbial. The sulfanilamides may be used to resist pathogens that infect the urinary tract and other areas of the body. For example, both sulfamethoxazole in combination with trimethoprim, and sulfadiazine may be useful in the treatment of urinary tract infections (UTIs). Sulfacetamide and its sodium salt are used in treatments for bacterial eye infections and also as a lotion used for the treatment of acne and dermatitis. Sulfaguanidine is a veterinary anti-bacterial used for intestinal infections. Thus, adding sulfanilamides, and their derivatives to a surface of the medical device and/or to the hydrophilic coating may help prevent contamination of the medical device and/or the coating by inhibiting the proliferation of pathogens such as bacteria, and microbes.

## Claims

1. A medical device comprising:
a surface; and
an antimicrobial comprising at least one of a sulfanilamide or a diaminodiphenyl sulfone, wherein the antimicrobial is grafted to the surface.

2. The medical device of claim 1, wherein the antimicrobial is sulfanilamide comprising: 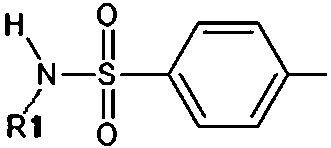 wherein R1 comprises one of hydrogen, acetyl groups, amido groups, guanidyl groups, 5 or 6 membered heterocyclic aromatic groups, methoxy-substituted heteroaromatic compounds and alkyl-substituted heteroaromatic compounds.

3. The medical device of claim 2, wherein the 5 or 6 membered heterocyclic aromatic groups contains one or more of nitrogen atom(s), oxygen atom(s) or sulfur atom(s).

4. The medical device of any one of claims 1-3, wherein the antimicrobial is sulfanilamide comprising one or more of: sulfanilamide, sulfapyridine, sulfamethoxydiazene, sulfathiazole, sulfacetamide, sulfadiazine, sulfadoxine, sulfamethizole, sulfacarbamide, sulfamethazine, sulfamethoxypyridazine, sulfamethoxazole, sulfafurazole, sulfaguanidine, sulfaisodimidine, sulfamethoxine, and sulfamoxole.

5. The medical device of any one of claims 1-5, wherein the device is a catheter.

6. The medical device of claim 5, wherein the catheter includes a shaft and antimicrobial is grafted to a surface of the shaft.

7. The medical device of any one of claims 1-6, wherein the device has a hydrophilic coating.

8. The medical device of claim 7, wherein the antimicrobial is grafted to the hydrophilic coating.

## Patentansprüche

1. Medizinische Vorrichtung, umfassend:
eine Oberfläche; und
ein antimikrobielles Mittel, umfassend mindestens eines von einem Sulfanilamid oder einem Diaminodiphenylsulfon, wobei das antimikrobielle Mittel auf die Oberfläche gepfropft ist.

2. Medizinische Vorrichtung nach Anspruch 1, wobei das antimikrobielle Mittel Sulfanilamid ist, umfassend: 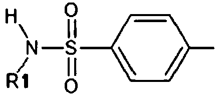 wobei R1 eines von Wasserstoff, Acetylgruppen, Amidogruppen, Guanidylgruppen, 5- oder 6-gliedrigen heterocyclischen aromatischen Gruppen, methoxysubstituierten heteroaromatischen Verbindungen und alkylsubstituierten heteroaromatischen Verbindungen umfasst.

3. Medizinische Vorrichtung nach Anspruch 2, wobei die 5- oder 6-gliedrigen heterocyclischen aromatischen Gruppen ein oder mehrere von Stickstoffatom(en), Sauerstoffatom(en) oder Schwefelatom(en) enthält.

4. Medizinische Vorrichtung nach einem der Ansprüche 1-3, wobei das antimikrobielle Mittel Sulfanilamid ist, umfassend ein oder mehrere von: Sulfanilamid, Sulfapyridin, Sulfamethoxydiazin, Sulfathiazol, Sulfacetamid, Sulfadiazin, Sulfadoxin, Sulfamethizol, Sulfacarbamid, Sulfamethazin, Sulfamethoxypyridazin, Sulfamethoxazol, Sulfafurazol, Sulfaguanidin, Sulfaisodimidin, Sulfamethoxin und Sulfamoxol.

5. Medizinische Vorrichtung nach einem der Ansprüche 1-5, wobei die Vorrichtung ein Katheter ist.

6. Medizinische Vorrichtung nach Anspruch 5, wobei der Katheter einen Schaft einschließt und antimikrobielles Mittel auf eine Oberfläche des Schafts gepfropft ist.

7. Medizinische Vorrichtung nach einem der Ansprüche 1-6, wobei die Vorrichtung eine hydrophile Beschichtung aufweist.

8. Medizinische Vorrichtung nach Anspruch 7, wobei das antimikrobielle Mittel auf die hydrophile Beschichtung gepfropft ist.

## Revendications

1. Dispositif médical, comprenant :
une surface ; et
un antimicrobien comprenant au moins l'un d'un sulfanilamide ou d'une diaminodiphénylsulfone, dans lequel l'antimicrobien est greffé à la surface.

2. Dispositif médical selon la revendication 1, dans lequel l'antimicrobien est le sulfanilamide, comprenant : 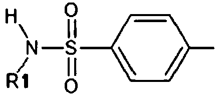 dans laquelle R1 comprend l'un parmi l'hydrogène, les groupes acétyle, les groupes amido, les groupes guanidyle, les groupes aromatiques hétérocycliques à 5 ou 6 chaînons, les composés hétéroaromatiques substitués par méthoxy et les composés hétéroaromatiques substitués par alkyle.

3. Dispositif médical selon la revendication 2, dans lequel les groupes aromatiques hétérocycliques à 5 ou 6 chaînons contiennent l'un ou plusieurs parmi un ou des atomes d'azote, un ou des atomes d'oxygène ou un ou des atomes de soufre.

4. Dispositif médical selon l'une quelconque des revendications 1 à 3, dans lequel l'antimicrobien est le sulfanilamide, comprenant l'un ou plusieurs : du sulfanilamide, de la sulfapyridine, du sulfaméthoxydiazène, du sulfathiazole, du sulfacétamide, de la sulfadiazine, de la sulfadoxine, du sulfaméthizole, du sulfacarbamide, de la sulfaméthazine, de la sulfaméthoxypyridazine, du sulfaméthoxazole, du sulfafurazole, de la sulfaguanidine, de la sulfaisodimidine, de la sulfaméthoxine et du sulfamoxole.

5. Dispositif médical selon l'une quelconque des revendications 1 à 5, dans lequel le dispositif est un cathéter.

6. Dispositif médical selon la revendication 5, dans lequel le cathéter comporte une tige et l'antimicrobien est greffé à une surface de la tige.

7. Dispositif médical selon l'une quelconque des revendications 1 à 6, dans lequel le dispositif présente un revêtement hydrophile.

8. Dispositif médical selon la revendication 7, dans lequel l'antimicrobien est greffé au revêtement hydrophile.
